Europäisches Patentamt

European Patent Office

Office européen des brevets

(19)

(11) Publication number: **0 287 395 B1**

## EUROPEAN PATENT SPECIFICATION

(12)

(45) Date of publication of patent specification: **22.12.93**

(51) Int. Cl.5: **C07K 7/08**, A61K 39/125, C12N 15/00

(21) Application number: **88303432.4**

(22) Date of filing: **15.04.88**

(54) **Human rhinovirus peptides.**

(30) Priority: **16.04.87 GB 8709274**

(43) Date of publication of application:
**19.10.88 Bulletin 88/42**

(45) Publication of the grant of the patent:
**22.12.93 Bulletin 93/51**

(84) Designated Contracting States:
**AT BE CH DE ES FR GB IT LI NL SE**

(56) References cited:
**EP-A- 0 192 175**

**JOURNAL GEN. VIROL., vol. 68, February 1987, SMG, GB;T. SKERN et al., pp. 315-323&NUM;**

**JOURNAL OF VIROLOGY, vol. 57, no. 1, January 1986, American Society for Microbiology; B. SHERRY et al., pp. 246-257&NUM;**

**NATURE, vol. 317, 12 September 1985; M.G. ROSSMANN et al., pp. 145-153&NUM;**

(73) Proprietor: **THE WELLCOME FOUNDATION LIMITED**
**Unicorn House**
**160 Euston Road**
**London NW1 2BP(GB)**

(72) Inventor: **Francis, Michael James**
**c/o The Wellcome Foundation Ltd.,**
**Langley Court**
**Beckenham, Kent BR3 3BS(GB)**
Inventor: **Clarke, Berwyn Ewart**
**c/o The Wellcome Foundation Ltd.,**
**Langley Court**
**Beckenham, Kent BR3 3BS(GB)**

(74) Representative: **Stott, Michael John et al**
**The Wellcome Foundation Limited**
**Group Patents & Agreements**
**Langley Court**
**Beckenham Kent BR3 3BS (GB)**

Rank Xerox (UK) Business Services
(3.10/3.6/3.3.1)

EP 0 287 395 B1

PROCEEDINGS OF THE NATL. ACADEMY OF SCIENCES USA, vol. 82, February 1985; P.L. CALLAHAN et al., pp. 732-736&NUM;

NUCLEIC ACIDS RESEARCH, vol. 13, no. 6, 1985; T. SKERN et al., pp. 2111-2116&NUM;

JOURNAL OF VIROLOGICAL METHODS, vol. 10, 1985, Elsevier Science Publishers B.V. (Biomedical Division); E.A. EMINI et al., pp. 163-170&NUM;

PROCEEDINGS OF THE NATL. ACADEMY OF SCIENCES USA, vol. 84, May 1987; M. DUECHLER et al., pp. 2605-2609&NUM;

JOURNAL GEN. VIROL., vol. 68, 1987, GB; M.J. FRANCIS et al., pp. 2687-2691&NUM;

**Description**

This invention relates to synthetic human rhinovirus (HRV) peptides, their preparation and their use as vaccines.

The HRV particle is composed of one molecule of single stranded RNA plus 60 copies of the structural proteins VP1 and VP3, 58 copies of VP2 and VP4 and 2 copies of their precursor VP0 (Medappa et al., 1971; Rueckert, 1976). The recent publication of the atomic structure of HRV type 14 (HRV14) (Rossmann et al. 1985) has provided detailed information on the location and structure of VP1, VP2 and VP3 as they appear on the surface of the intact virion.

The positions of amino acid substitution found in neutralization resistant mutants selected by a number of monoclonal antibodies have been used to identify four antigenic sites on the HRV14 particle (Sherry & Rueckert, 1985; Sherry et al., 1986). These sites have been referred to as NIm-IA, NIm-IB, NIm-II and NIm-III according to the viral protein on which they are chiefly located. By using a monoclonal antibody, a neutralising epitope on VP2 of HRV2 has also been mapped. This epitope includes amino acid residues from 153 to 164 (Skern et al., 1987). These twelve residues are, using the one-letter code:

GREVKAETRLNP.

According to Skern et al, analysis of the binding properties of proteins expressed from plasmids containing a trunkated gene of VP2 showed that the binding site of the monoclonal antibody was maintained provided deletions from the carboxy end did not extend further than amino acid 164. No binding was observed as soon as the deletion reached amino acid 153. However, the lack of a suitable restriction site in the sequence between the N-terminus of VP2 and the region of VP2 of interest precluded a definition of the N-terminal boundary of the binding site of the monoclonal antibody.

The antigenic sites which have been identified for HRV so far have all been predicted from work on the virus particle. No work has been reported using synthetic peptides. However, there has been increasing interest in developing synthetic peptides for use as vaccines against other picornaviruses such as foot-and-mouth virus and poliovirus.

We have now produced synthetic peptides to six predicted immunogenic determinants on HRV2. Four of these peptides were found to react with rabbit anti-HRV2 serum. Of these four, three produced anti-serum which reacted well with virus particles in an indirect ELISA and by Western blotting. However anti-serum to one of the peptides reacted with virus in a sandwich ELISA and an immunoprecipitation test, and neutralised its infectivity in vitro. This peptide is:

VKAETRLNPDLQPTEC.

In other words, the peptide corresponds to amino acid residues 156 to 170 of VP2 of HRV2. It has also a non-natural carboxy-terminal cysteine residue. Only nine of the amino acid residues of the epitope proposed by Skern at al are present in the peptide. Synthetic peptides comprising this nonapeptide sequence are thus capable of raising neutralising antibody to HRV2.

Accordingly, the present invention provides peptides suitable for use as vaccines, which peptides are composed of up to 50 amino acid residues and present an epitope comprising amino acid residues 156 to 165 of VP2 of HRV2 or equivalent amino acid residues of another HRV. These amino acids may be replaced by other amino acids which do not affect the antigenicity of the peptide. The peptides may be provided as a conjugate linked to a physiologically acceptable carrier. Alternatively, the peptides may be presented in the form fusion proteins comprising the sequence of a said peptide linked to a carrier sequence.

The peptides of the invention comprise a at least a minimum antigenically effective sequence, which for HRV2 is:

VKAETRLNPD.

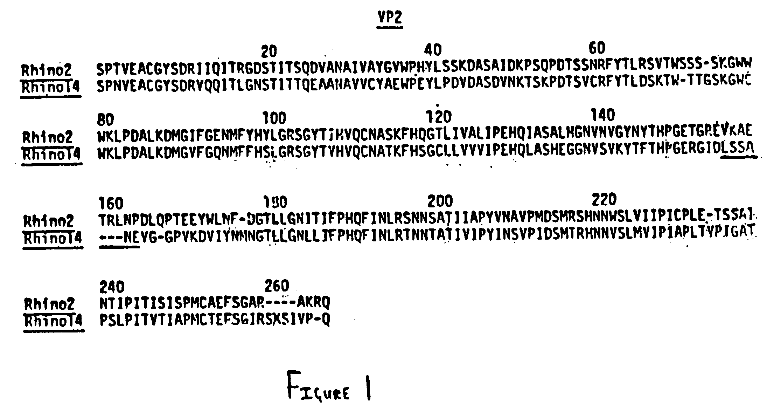

The term "minimum antigenically effective sequence" is used herein to mean amino acids 156 to 165 of VP2 of HRV2 and equivalent sequences of other HRV's. Counterpart VP2 residues of another HRV serotype may be used in place of residues 156 to 165 of VP2 of HRV2. The counterpart residues can readily be determined by lining up the VP2 sequence of another HRV with the VP2 sequence of HRV2. This is a straightforward matter because of the homology between VP2 sequences of different types of HRV. Figure 1 of the accompanying drawings illustrates this. Here, the VP2 sequences of HRV2 and HRV14 have been lined up. It can be seen that residues 156 to 162 of HRV14 correspond to residues 156 to 165 of HRV2. These HRV14 residues, underlined in the Figure, are:

LSSANEV.

One or more amino acids of the defined minimum antigenically effective sequences may be replaced by one or more other amino acids which do not affect the antigenicity of the epitope. Consequently, an amino acid may be replaced by another one which preserves the physicochemical character of the original

3

epitope, i.e. in terms of charge density, hydrophilicity/hydrophobicity, size and configuration, and hence preserve the immunological structure. For example, A may be replaced by G or vice versa; V by A, L or G; K by R; S by T or vice versa; E by D or vice versa; and Q by N or vice versa. As mentioned above, this is provided that the changes do not affect the antigenicity of the epitope.

In its simplest form, a peptide may consist of amino acid residues 156 to 165 of VP2 of HRV2 or the corresponding amino acid residues of another HRV. For HRV2 and HRV 14 respectively, these are the peptides:

VKAETRLNPD and LSSANEV.

Alternatively, longer peptides presenting the minimum antigenically effective epitope may be provided. Further amino acid residues may be added to one or both ends of the minimum antigenically effective sequence. Thus, a peptide of up to 50, for example of up to 20, amino acid residues can be built up. One, two, three or more amino acid residues may be added to either terminus. In one embodiment, it is only to the carboxy-terminus that extra amino acid residues are added. Up to 30, for example up to 15 or up to 6, further amino acid residues may be independently provided at either or each terminus of the minimum antigenically effective epitope.

The amino acid residues which are added to the minimum antigenically effective sequence derivable from a particular HRV can be the ones provided in corresponding positions in the VP2 sequence for that HRV. A preferred peptide built up in this way comprises amino acids 156 to 170 of VP2 of HRV2 or the corresponding sequence from HRV14. These sequences are:

VKAETRLNPDLQPTE and LSSANEVGGPVK

Further, longer peptides may also comprise multiple copies of the minimum antigenically effective epitope or of sequences as defined above incorporating this epitope. Also, a cysteine (C) residue may be added to either or both terminals of the present peptides. In particular, a C residue may be added to the carboxy-terminus alone. This is added to facilitate carrier coupling and/or to enhance the immunogenicity of a peptide.

The peptide may be linked to a carrier in order to create a conjugate which is immunogenically active. Any appropriate physiologically acceptable carrier may be employed. For example, the carrier may be bovine serum albumin, thyroglobulin, ovalbumin or keyhole limpet hemocyanin. The peptide may be linked to tetanus toxoid and/or diptheria toxoid thus providing both an immunogen and a multivalent vaccine at the same time.

Rather than link a carrier sequence to a peptide in this way, a peptide may be prepared which itself incorporates a carrier sequence for the VP2 epitope. The minimum antigenically effective sequence or the sequence of a longer peptide incorporating the minimum antigenically effective sequence may be linked to or provided within the carrier sequence. A heterologous carrier sequence is provided. By this is meant that the total sequence of the peptide, for example a fusion protein, comprising the minimum antigenically effective sequence and the carrier sequence does not correspond to all or part of the sequence of VP2 of a particular HRV. The carrier sequence may not be a HRV sequence at all. The carrier protein is chosen so that the resultant product is physiologically acceptable.

A peptide of the invention is a synthetic peptide. It may be prepared by chemical synthesis from single amino acids and/or preformed peptides of two or more amino acid residues. Solid phase or solution methods may be employed. Alternatively, the peptides may be prepared by recombinant DNA methodologies. Thus, a DNA sequence encoding a minimum antigenically effective sequence or a longer sequence incorporating this sequence is provided. An expression vector is prepared which incorporates the DNA sequence and which is capable of expressing the peptide when provided in a suitable host. The DNA sequence is located between translation start and stop signals in the vector. Appropriate transcriptional control elements are also provided, in particular a promoter for the DNA sequence and a transcriptional termination site. The DNA sequence is provided in the correct frame such as to enable expression of the peptide to occur in a host compatible with the vector.

Any appropriate host-vector system may be employed. The vector may be a plasmid. In that event, a bacterial or yeast host may be used. Alternatively, the vector may be a viral vector. This may be used to transfect cells of a mammalian cell line in order to cause peptide expression.

The viral vector may be a recombinant vaccinia virus. Typically a DNA sequence encoding a minimum antigenically effective sequence or a longer sequence incorporating this sequence is inserted into a plasmid vector downstream of a vaccinia virus promoter and flanked by vaccinia thymidine kinase (TK) sequences. The resultant recombination vector is introduced into cells invected with vaccinia virus. As a result of homologous recombination, a TK$^-$ recombinant vaccinia virus is generated which expresses the peptide.

One example of the use of recombinant DNA technology to present the minimum antigenically effective epitope as part of a fusion protein is as follows. A DNA sequence can be provided encoding a fusion protein comprising influenza virus haemagglutinin (HA) at a site of which normally occupied by a natural antigenic epitope thereof the minimum antigenically effective epitope is provided. In other words all or part of a natural antigenic epitope of HA may be replaced by a minimum antigenically effective epitope or a longer sequence incorporating the minimum antigenically effective sequence.

For expression of the fusion protein, the DNA sequence is incorporated in an expression vector. The DNA sequence is incorporated in a vector such that the vector, when provided in a eucaryotic host, is capable of expressing the fusion protein. A eucaryotic host is required for correct glycosylation of the HA. The vector may be a viral vector which incorporates the DNA sequence such that the fusion protein is expressed in cells infected with the vector. A vaccine may comprise such a viral vector and a physiologically acceptable carrier or diluent. In such an instance, the viral vector preferably is a recombinant vaccinia virus which incorporates the DNA sequence. Alternatively, a vaccine may comprise the fusion protein and a physiologically acceptable carrier or diluent.

Influenza virus HA is an integral membrane protein. When expressed in recombinant vaccinia virus infected cells the HA fusion protein is glycosylated normally and transported to the cell surface through which it protrudes. Here, it presents the minimum antigenically effective epitope on the outside of the cell surface. Typically, the minimum antigenically effective epitope is provided at antigenic site A of HA.

Another example of the use of recombinant DNA technology to prepare a fusion protein to present the minimum antigenically effective epitope to the immune system is to produce a fusion protein comprising HBcAg to the amino terminus of which is linked a minimum antigenically effective epitope or a longer sequence incorporating the minimum antigenically effective epitope. This fusion protein may be expressed in the same way as the HA fusion protein except that any compatible host system may be employed, not simply a eucaryotic one. Also, it is only the fusion protein which may be used as a vaccine as HBcAg is not a surface protein. The minimum antigenically effective epitope may be fused directly to the HBcAg. Alternatively, the epitope may be fused to the HBcAg via an intervening linker. Such a linker may be composed of one or more, for example up to ten, amino acid residues.

The peptides of the invention can raise neutralising antibody. They therefore may be used as vaccines for humans. Vaccination is achieved by administering to a patient an effective amount of a peptide. An oral route or a parenteral route such as sub-cutaneously, intravenously or intramuscularly may be adopted. Typically, a peptide is administered in an amount of 1 to 1,000 $\mu$g per dose, more preferably 10 to 100 $\mu$g per dose, by either the oral or the parenteral route. In the case where a viral vector capable of expressing a HA fusion protein is utilised as a vaccine, the routes of administration and amount of virus given are the same. For a recombinant vaccinia virus, this may be administered by the dermal route.

For vaccination purposes, a peptide is typically formulated with a pharmaceutically acceptable carrier or diluent. Conventional formulations, carriers, adjuvants and diluents may be employed. These will of course be determined by the route of administration.

The vaccines administered to patients preferably will include not just one peptide incorporating a minimum antigenically effective epitope but also other antigens. These may be in the form of peptides incorporating a minimum antigenically effective epitope of a different type of HRV and/or other epitopes of HRV.

The following Examples illustrate the invention. In the accompanying drawings:

Figure 1 shows the VP2 sequences for HRV2 and HRV14;

Figure 2 is the Fast Atom Bombardment Mass Spectrum (FABMS) of the molecular ion region of peptide 4 (Example 1);

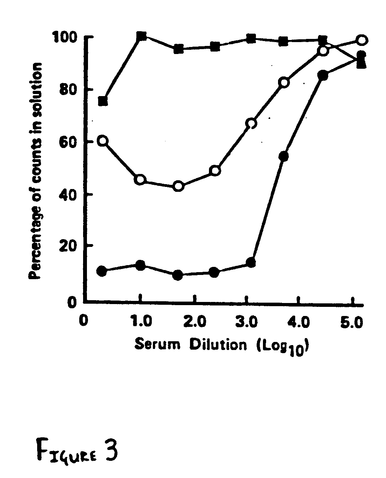

Figure 3 shows the results of immunoprecipitation of purified HRV2 by anti-peptide sera to the minimum antigenically effective sequence (Example 2); and

Figure 4 shows the results of Western blot analysis on anti-HRV2 peptide sera (Example 2).

Example 1: Preparation of peptides

By reference to a computer graphic model of HRV14 (generated by Dr. D. Stuart, Dept of Molecular Biophysics, Oxford University, GB using coordinates provided by Dr. M.G. Rossmann) and by sequence alignment of HRV14 with HRV2, another serotype of the virus (Stanway et al., 1984; Skern et al., 1985; Callahan et al., 1985), we selected a number of potentially important immunogenic sites on HRV2 (three from VP1, one from VP2 and two from VP3) for assessment using synthetic peptides. The sequences and locations of the HRV2 peptides produced are given in Table 1. Synthesis was carried out using an adaption of the Merrifield (1963) technique described by Houghten (1985). Each peptide has an additional non-natural cysteine residue at its C-terminus to facilitate coupling to keyhole limpet haemocyanin (KLH).

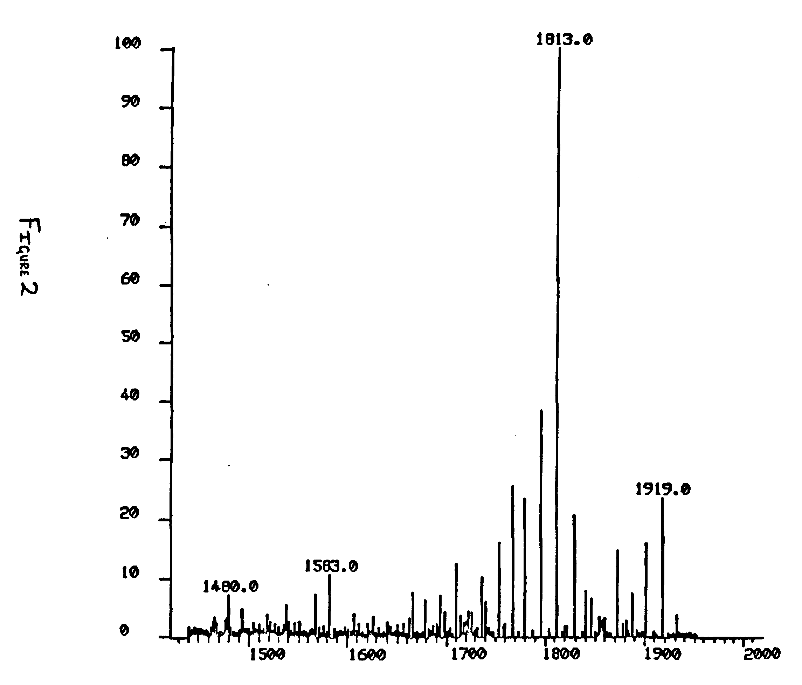

A Fast Atom Bombardment Mass Spectrum of the molecular ion region of peptide 4 (molecular weight 1811.922) is shown in Figure 2. The FAB spectrum shows a $(M + H)^+$ ion for the expected compound at M/Z 1813, thus confirming the proposed molecular weight.

Example 2: Tests of the peptides

The peptides were initially tested in vitro for their reactivity with rabbit anti-HRV2 serum using an indirect ELISA, in which a range of peptide concentrations bound to the solid phase were screened with an excess of anti-serum. This technique identified four reactive peptides, two from VP1, one from VP2 and one from VP3 (Table 1).

These four peptides were therefore selected for immunisation after coupling to KLH by the MBS method (Liu et al., 1979). Pairs of rabbits were inoculated intramuscularly with 500 ug of each peptide emulsified with complete Freund's adjuvant and reinoculated subcutaneously, 42 days later, with the same dose emulsified with incomplete Freund's adjuvant. Serum samples were monitored at 14 day intervals for anti-peptide activity. All the rabbits produced anti-peptide antibodies that were detectable within 14 days of the first inoculation, and in the final bleeds collected 28 days after revaccination the anti-peptide activity as measured by indirect ELISA ranged for 3.7 $\log_{10}$ to 4.7 $\log_{10}$ (Table 2).

The anti-peptide sera were subsequently examined for anti-HRV2 activity using several different assay systems; namely indirect ELISA, sandwich ELISA immunoprecipitation and neutralisation (Table 2). All the antisera reacted with HRV2 in an indirect ELISA, with antisera to peptides 3, 4 and 6 giving significantly higher titres (3.1 to 5.2 $\log_{10}$) than antisera to peptide 2 (1.5 to 1.8 $\log_{10}$). However, only the antiserum from rabbit number 5 inoculated with peptide 4 from VP2 reacted well in a sandwich ELISA.

The difference in results with the two assays is probably related to the degree of distortion of the virus particles induced by the method of binding to the solid phase (McCullough et al., 1985). Direct binding to a plastic surface, as in the indirect ELISA, causes a distortion of another picornavirus, foot-and-mouth disease virus, exposing antigenic sites not normally accessible to peptide antibodies. The sandwich ELISA imposes less physical alteration of virus particles since they are trapped by immobilised antibody.

Since both ELISA techniques may distort the virus, to a greater or less extent, sera were also allowed to react in solution with purified $^{35}$S-methionine labelled HRV2 particles and precipitated using staphylococcus A ghosts. Only the sera to peptide 4 showed any significant precipitation of the virus (Table 2). However, the extent of precipitation observed was significantly different between the two rabbit sera tested (Fig 1). The anti-serum from rabbit number 5 (•—•) precipitated >90% of the labelled virus while that from rabbit number 6 (○—○) precipitated only 58%, in the some assay (•—■ is normal rabbit antiserum). This result is probably due to a difference in the overall functional affinities of the two antisera for native rhinovirus particles. The antiserum that was more active in precipitating the virus was also the one that reacted in the sandwich ELISA.

All the peptide antisera were also tested for their ability to neutralise the virus. Undiluted and a 1:4 dilution of each anti-serum were incubated with a range of virus dilutions and the difference in $\log_{10}$ titre obtained with virus plus normal rabbit serum and virus plus anti-peptide serum was taken as the neuteralisation index. Using this technique only the antiserum to peptide 4 gave any neutralising activity, with rabbit number 5 anti-serum having significantly greater activity than that from rabbit number 6 (Table 2). It was this antiserum that reacted best in the sandwich ELISA and immunoprecipitation tests. Since both sera had similar levels of anti-peptide activity, the differences observed in the neutralising antibody assay must be due to qualitative differences in the antibody populations with respect to the conformation of the epitope they recognise.

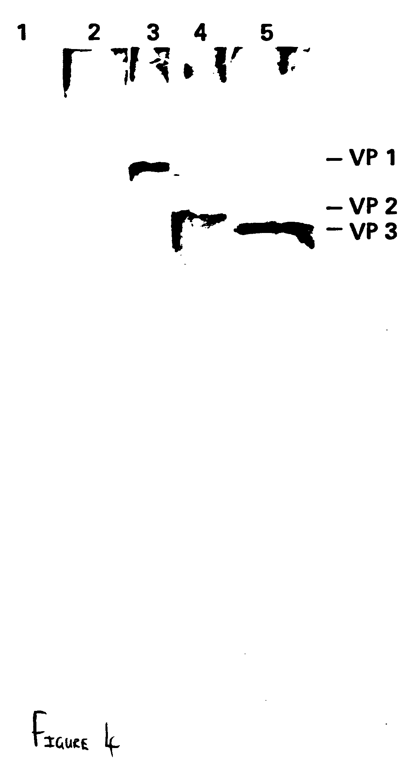

Finally, Western blotting was performed to determine whether the peptide anti-sera recognised isolated viral proteins in a specific manner. In contrast to the polyclonal anti-HRV2 serum (lane 1), which failed to react, each of the anti-peptide sera reacted with the corresponding virus protein, with antisera to peptides 3 (lane 3), 4 (lane 4) and 6 (lane 5) having the highest reactivity (Fig. 4; lane 2 is antisera to peptide 2). These observations support the indirect ELISA results. Furthermore, the monoclonal antibody described by Skern et al. (1987) covering our peptide 4 site also reacted in Western blots. In general the reactions were protein specific although peptide 3 antiserum cross-reacted with VP2 and VP3, and peptide 4 antiserum cross-reacted with VP1 to a limited extent. However, the level of cross-reactivity is so low that the antisera will provide useful marker reagents for the virus proteins.

Example 3: HRV 14 peptide

A peptide for the sequence LSSANEVGGPVKC was synthesised using an adaption of the Merrifield (1963) technique described by Houghten (1985). An non-natural cystein residue was provided at the C-terminus of the peptide. The peptide was coupled to KLH (5 mg peptide/3.2 mg KLH).

The activity of the peptide was examined. On day 0 500 $\mu$g of the peptide, coupled to KLH, was inoculated intramuscularly (i.m.) into two rabbits using complete Freund's adjuvant. On day 42 another 500 $\mu$g of the peptide, coupled to KLH, was inoculated i.m. into each rabbit using incomplete Freund's adjuvant. Both rabbits were bled on day 84. Anti-peptide and anti-HRV14 activity was measured by indirect ELISA. The results are shown in Table 3 below.

## TABLE 3

| | Indirect ELISA (50% OD Max) | | Neutralisation |
|---|---|---|---|
| | Peptide | HRV14 | Index |
| Rabbit No. 1 | 3.7* | 3.8 | 0.7 |
| Rabbit No. 2 | 4.1 | >4.0 | 1.7 |

*$\log_{10}$

REFERENCES

Callahan et al. (1985) Proceedings of the National Academy of Sciences, U.S.A. 82, 732-736
Houghten (1985) Proceedings of the National Academy of Sciences, U.S.A. 82, 5131-5135
Liu et al (1979) Biochemistry 18, 690-697
McCullough et al (1985) Journal of Immunological Methods 82, 91-100
Medappa et al (1971) Virology 44, 259-270
Merrifield (1963) Journal of the American Chemical Society 85, 2149-2154
Rossmann et al (1985) Nature, London 317, 145-153
Rueckert (1976) In Comprehensive Virology, Vol 6, pp. 131-213. Edited by H. Fraenkel-Conrat & R.R. Wagner, Plenum Press, New York
Sherry and Rueckert (1985) Journal of Virology 53, 137-143
Sherry et al (1986) Journal of Virology 57, 246-257
Skern et al (1985) Nucleic Acids Research 13, 2111-2126
Skern et al (1987) Journal of General Virology 68, 315-323
Stanway et al (1984) Nucleic Acids Research 12, 7859-7875

Table 1 - Human rhinovirus type 2 peptides: location, sequence and reactivity with anti-HRV2 serum.

| Peptide number | Viral protein | Location[1] | Amino acid sequence | Reactivity with anti-HRV2 sera[*] (INDIRECT ELISA) |
|---|---|---|---|---|
| 1 | VP1 | NIm-IA/ NIm-IB | KLEVTLANYNKENFTC | - |
| 2 | VP1 | Two parts of NIm-IB | KHIHKDIGHC[‡] | ++ |
| 3 | VP1 | Carboxy terminus. Part of NIm-III | IQTAIVTRPIITTAGPSDMYC | ++++ |
| 4 | VP2 | NIm-II | VKAETRLNPDLQPTEC | +++ |
| 5 | VP3 | NIm-III | VLQSSINAPDKC | - |
| 6 | VP3 | Carboxy terminus. Edge of canyon[†] | FCLRMARDTNLHLQSGAIAQC | ++ |

* Concentration of peptide reactive with antiserum: ++++ 0.01 µg/ml, +++ 0.1 µg/ml, ++ 1 µg/ml, + 10 µg/ml and - <100 µg/ml.

† Rossmann et al., 1985.

‡ This sequence is not contiguous on the VP1 primary structure.

1 The location of each peptides 1 to 5 is identified by reference to the corresponding sequence of HRV14.

Table 2 - Analysis of rabbit anti-HRV2 peptide sera.

| Peptide number | Viral protein | Rabbit number | Indirect ELISA* | | Sandwich ELISA* | Immuno-precipitation† | Neutralization index ($Log_{10}$) | |
|---|---|---|---|---|---|---|---|---|
| | | | Anti-peptide | Anti-virus | | | Undiluted | 1:4 dilution |
| 2 | VP1 | 1 | 3.7 | 1.5 | <1.0 | <0.3 | 0 | 0 |
| | | 2 | 4.2 | 1.8 | <1.0 | <0.3 | 0 | 0 |
| 3 | VP1 | 3 | 3.8 | 4.9 | <1.0 | <0.3 | 0 | 0 |
| | | 4 | 4.3 | 3.9 | <1.0 | <0.3 | 0.1 | 0 |
| | | 5 | 4.7 | 5.2 | 1.6 | 3.7 | 2.4 | 1.9 |
| 4 | VP2 | 6 | 4.6 | 4.2 | <1.0 | 2.5 | 0.6 | 0 |
| | | 7 | 4.3 | 3.1 | <1.0 | <0.3 | 0 | 0 |
| 6 | VP3 | 8 | 4.0 | 3.8 | <1.0 | <0.3 | 0 | 0 |

* $Log_{10}$ dilution giving 50% O.D. maximum.
† $Log_{10}$ dilution precipitating 50% of counts.

**Claims**

**Claims for the following Contracting States : AT, BE, CH, DE, FR, GB, IT, LI, NL, SE**

1. A synthetic peptide suitable for use as a vaccine against a human rhinovirus (HRV), which peptide is composed of up to 50 amino acid residues and presents an epitope comprising amino acid residues 156 to 165 of VP2 of HRV type 2 or equivalent amino acid residues of another HRV, optionally one or more of the said residues being replaced by one or more other amino acid residues which do not affect the antigenicity of the peptide.

2. A peptide according to claim 1, which is composed of up to 20 amino acid residues.

3. A peptide according to claim 1, which is composed of the amino acid residues of the said epitope and up to 15 extra amino acids added to the C-terminus thereof.

4. A peptide according to any one of the preceding claims, which comprises the sequence:
   VKAETRLNPDLQPTE or
   LSSANEVGGPVK

5. A peptide according to any one of the preceding claims, comprising additionally a non-natural cysteine residue at either or both terminals.

6. A fusion protein comprising the sequence of a peptide as claimed in any one of the preceding claims, linked to a heterologous carrier sequence.

7. A fusion protein according to claim 6, comprising hepatitis B core antigen (HBcAg) to the amino terminus of which is linked the sequence of a said peptide.

8. A process for the preparation of a synthetic peptide as claimed in any one of claims 1 to 5, which process comprises chemically synthesising the said peptide from single amino acids and/or preformed peptides of two or more amino acid residues.

9. A process for the preparation of a synthetic peptide as claimed in any one of claims 1 to 5 or a fusion protein as claimed in claim 6 or 7, which process comprises:
   (i) preparing an expression vector which incorporates a DNA sequence encoding the said peptide or fusion protein and which is capable of expressing the said peptide or fusion protein when provided in a suitable host, and
   (ii) providing the said expression vector in the said host such as to enable expression of the peptide or fusion protein to occur.

10. A conjugate suitable for use as a vaccine against a HRV, comprising a peptide as defined in any one of claims 1 to 5 linked to a physiologically acceptable carrier.

11. A vaccine suitable for use against a HRV, which vaccine comprises a peptide as defined in any one of claims 1 to 5, a fusion protein as defined in claim 6 or 7, or a conjugate as defined in claim 10 formulated with a pharmaceutically acceptable carrier or diluent.

**Claims for the following Contracting State : ES**

1. A process for the preparation of a synthetic peptide suitable for use as a vaccine against a human rhinovirus (HRV), which peptide is composed of up to 50 amino acids and presents an epitope comprising amino acid residues 156 to 165 of VP2 of HRV type 2 or equivalent amino acid residues of another HRV, optionally one or more of the said residues being replaced by one or more other amino acid residues which do not affect the antigenicity of the peptide; which process comprises chemically synthesising the said peptide from single amino acids and/or preformed peptides of two or more amino acid residues.

2. A process according to claim 1, wherein the said peptide is composed of up to 20 amino acid residues.

3. A process according to claim 1, wherein the said peptide is composed of the amino acid residues of the said epitope and up to 15 extra amino acids added to the C-terminus thereof.

4. A process according to any one of the preceding claims, wherein the said peptide comprises additionally a non-natural cysteine residue at either or both terminals.

10

**5.** A process according to any one of the preceding claims, wherein the said peptide incorporates a heterologous carrier sequence for the said epitope.

**6.** A process according to any one of claims 1 to 4, further comprising linking the peptide thus-produced to a physiologically acceptable carrier therefor.

**7.** A process for the preparation of a synthetic peptide suitable for use as a vaccine against a human rhinovirus (HRV), which peptide is composed of up to 50 amino acids and presents an epitope comprising amino acid residues 156 to 165 of VP2 of HRV type 2 or equivalent amino acid residues of another HRV, optionally one or more of the acid residues being replaced by one or more other amino acid residue which do not affect the antigenicity of the peptide; or of a fusion protein in which the said peptide is linked to a heterologous carrier sequence therefor; which process comprises:
  (i) preparing an expression vector which incorporates a DNA sequence encoding the said peptide or fusion protein and which is capable of expressing the said peptide or fusion protein when provided in a suitable host, and
  (ii) providing the said expression vector in the said host such as to enable expression of the peptide or fusion protein to occur.

**8.** A process according to claim 7, wherein the said peptide is composed of up to 20 amino acid residues.

**9.** A process according to claim 7, wherein the said peptide is composed of the amino acid residues of the said epitope and up to 15 extra amino acids added to the C-terminus thereof.

**10.** A process according to any one of claims 7 to 9, wherein the said carrier sequence is hepatitis B core antigen (HBcAg) to the amino terminus of which is linked the sequence of a said peptide.

**11.** A process for the preparation of a vaccine, which process comprises formulating with a pharmaceuticaly acceptable carrier or diluent the product of the process of any one of the preceding claims.

**Patentansprüche**
**Patentansprüch für folgende Vertragsstaaten : AT, BE, CH, DE, FR, GB, IT, LI, NL, SE**

**1.** Synthetisches Peptid, welches als Impfstoff gegen Human-Rhinovirus (HRV) geeignet ist, welches Peptid aus bis zu 50 Aminosäureresten aufgebaut ist und ein Epitop aufweist, welches die Aminosäurereste 156 bis 165 des VP2 von HRV Typ 2 oder äquivalente Aminosäurereste eines anderen HRV umfaßt, wobei gegebenenfalls einer oder mehrere dieser Reste durch einen oder mehrere andere Aminosäurereste ersetzt ist, welche die Antigenizität des Peptids nicht beeinträchtigen.

**2.** Peptid nach Anspruch 1, welches aus bis zu 20 Aminosäureresten aufgebaut ist.

**3.** Peptid nach Anspruch 1, welches aus den Aminosäureresten des genannten Epitops und bis zu 15 weiteren Aminosäuren, die an dessen C-Ende gebunden sind, aufgebaut ist.

**4.** Peptid nach einem der vorhergehenden Ansprüche, welches die Sequenz
    VKAETRLNPDLQPTE oder
    LSSANEVGGPVK
umfaßt.

**5.** Peptid nach einem der vorhergehenden Ansprüche, enthaltend zusätzlich an entweder einem oder beiden Enden einen nicht-natürlichen Cysteinrest.

**6.** Fusionsprotein enthaltend die Sequenz eines Peptids nach einem der vorhergehenden Ansprüche, gebunden an eine heterologe Trägersequenz.

**7.** Fusionsprotein nach Anspruch 6, umfassend das Hepatitis B-Kern-Antigen (HBcAg), an dessen Amino-Ende die Sequenz eines solchen Peptids gebunden ist.

**8.** Verfahren zur Herstellung eines synthetischen Peptids nach einem der Ansprüche 1 bis 5, welches Verfahren darin besteht, das Peptid aus einzelnen Aminosäuren und/oder vorgebildeten Peptiden aus zwei oder mehreren Aminosäureresten chemisch zu synthetisieren.

**9.** Verfahren zur Herstellung eines synthetischen Peptids nach einem der Ansprüche 1 bis 5 oder eines Fusionsproteins nach Anspruch 6 oder 7, welches Verfahren darin besteht:

(i) einen Expressionsvektor herzustellen, der eine DNA-Sequenz enthält, die für das Peptid oder das Fusionsprotein codiert und dazu geeignet ist, das Peptid oder das Fusionsprotein zu exprimieren, wenn er in einen geeigneten Wirt eingebracht wird, und

(ii) Einführen des Expressionsvektors in den Wirt, um die Expression des Peptids oder des Fusionsproteins zu ermöglichen.

**10.** Konjugat geeignet zur Verwendung als Impfstoff gegen einen HRV, enthaltend ein Peptid nach einem der Ansprüche 1 bis 5, gebunden an einen physiologisch annehmbaren Träger.

**11.** Impfstoff geeignet zur Verwendung gegen HRV, welcher Impfstoff ein Peptid, wie es in einem der Ansprüche 1 bis 5 definiert ist, ein Fusionsprotein, wie es in Anspruch 6 oder 7 definiert ist, oder ein Konjugat, wie es in Anspruch 10 definiert ist, umfaßt, in Kombination mit einem pharmazeutisch annehmbaren Träger oder Verdünnungsmittel.

**Patentansprüche für folgenden Vertragsstaat : ES**

**1.** Verfahren zur Herstellung eines synthetischen Peptids, welches als Impfstoff gegen Human-Rhinovirus (HRV) geeignet ist, welches Peptid aus bis zu 50 Aminosäureresten aufgebaut ist und ein Epitop aufweist, welches die Aminosäurereste 156 bis 165 des VP2 von HRV Typ 2 oder äquivalente Aminosäurereste eines anderen HRV umfaßt, wobei gegebenenfalls einer oder mehrere dieser Reste durch einen oder mehrere andere Aminosäurereste ersetzt ist, welche die Antigenizität des Peptids nicht beeinträchtigen, welches Verfahren darin besteht, das Peptid aus einzelnen Aminosäuren und/oder vorgebildeten Peptiden aus zwei oder mehreren Aminosäureresten chemisch zu synthetisieren.

**2.** Verfahren nach Anspruch 1, worin das Peptid aus bis zu 20 Aminosäureresten aufgebaut ist.

**3.** Verfahren nach Anspruch 1, worin das Peptid aus den Aminosäureresten des Epitops und bis zu 15 weiteren Aminosäuren, die an dessen C-Ende gebunden sind, aufgebaut ist.

**4.** Verfahren nach einem der vorhergehenden Ansprüche, worin das Peptid zusätzlich einen an entweder eines oder beide Enden gebundenen nicht-natürlichen Cysteinrest umfaßt.

**5.** Verfahren nach einem der vorhergehenden Ansprüche, worin das Peptid eine heterologe Trägersequenz für das Epitop umfaßt.

**6.** Verfahren nach einem der Ansprüche 1 bis 4, umfassen das Verbinden des in dieser Weise gebildeten Peptids mit einem dafür geeigneten physiologisch annehmbaren Träger.

**7.** Verfahren zur Herstellung eines synthetischen Peptids, welches zur Verwendung als Impfstoff gegen einen Human-Rhinovirus (HRV) geeignet ist, welches Peptid aus bis zu 50 Aminosäuren aufgebaut ist und ein Epitop umfaßt, welches die Aminosäurereste 156 bis 165 des VP2 von HRV Typ 2 oder äquivalente Aminosäurereste eines anderen HRV umfaßt, wobei gegebenenfalls einer oder mehrere der Säurereste durch einen oder mehrere Aminosäurereste ersetzt sind, welche die Antigenizität des Peptids nicht beeinträchtigen; oder aus einem Fusionsprotein aufgebaut ist, in dem das Peptid an eine heterologe Trägersequenz dafür gebunden ist, welches Verfahren darin besteht:

(i) einen Expressionsvektor herzustellen, welcher eine DNA-Sequenz enthält, die für das Peptid oder das Fusionsprotein codiert und welche dazu geeignet ist, das Peptid oder Fusionsprotein zu exprimieren, wenn er in einen geeigneten Wirt eingebracht wird, und

(ii) Einbringen des Expressionsvektors in den Wirt, um die Expression des Peptids oder des Fusionsproteins zu ermöglichen.

**8.** Verfahren nach Anspruch 7, worin das Peptid aus bis zu 20 Aminosäureresten aufgebaut ist.

**9.** Verfahren nach Anspruch 7, worin das Peptid aus den Aminosäureresten des Epitops und bis zu 15 weiteren Aminosäuren, die an dessen C-Ende gebunden sind, aufgebaut ist.

**10.** Verfahren nach einem der Ansprüche 7 bis 9, worin die Trägersequenz das Hepatitis B-Kern-Antigen (HBcAg) ist, an dessen Amino-Ende die Sequenz eines solchen Peptids gebunden ist.

**11.** Verfahren zur Herstellung eines Impfstoffs, welches Verfahren darin besteht, das Produkt des Verfahrens nach einem der vorhergehenden Ansprüche mit einem pharmazeutisch annehmbaren Trägermaterial oder Verdünnungsmittel zu formulieren.

**Revendications**
**Revendications pour les Etats contractants suivants : AT, BE, CH, DE, FR, GB, IT, LI, NL, SE**

**1.** Peptide synthétique approprié à l'utilisation en tant que vaccin contre un rhinovirus humain (RVH), lequel peptide est composé de jusqu'à 50 résidus acide aminé et présente un épitope comprenant les résidus acide aminé 156 à 165 de VP2 du RVH de type 2 ou des résidus acide aminé équivalents d'un autre RVH, facultativement un ou plusieurs de ces résidus peuvent être remplacés par un ou plusieurs autres résidus acide aminé qui n'affectent pas la fonction antigénique du peptide.

**2.** Peptide suivant la revendication 1, qui est composé de jusqu'à 20 résidus acide aminé.

**3.** Peptide suivant la revendication 1, qui est composé des résidus acide aminé de l'épitope et jusqu'à 15 acides aminés supplémentaires ajoutés à l'extrémité C de celui-ci.

**4.** Peptide suivant l'une quelconque des revendications précédentes, qui comprend les séquences
    VKAETRLNPDLQPTE ou LSSANEVGGPVK.

**5.** Peptide suivant l'une quelconque des revendications précédentes, comprenant un résidu cystéine non naturel additionnel à l'une ou aux deux extrémités.

**6.** Protéine de fusion comprenant la séquence d'un peptide tel que revendiqué dans l'une quelconque des revendications précédentes, liée a une séquence porteur hétérologue.

**7.** Protéine de fusion suivant la revendication 6, comprenant l'antigène de l'hépatite B (HBcAg) à l'extrémité amino duquel est liée la séquence dudit peptide.

**8.** Procédé pour la préparation d'un peptide synthétique suivant l'une quelconque des revendications 1 à 5, lequel procédé comprend la synthèse chimique du peptide à partir des acides aminés uniques et/ou de peptides préformés de deux ou plusieurs résidus acide aminé.

**9.** Procédé pour la préparation d'un peptide synthétique suivant l'une quelconque des revendications 1 à 5, ou d'une protéine de fusion suivant la revendication 6 ou 7, lequel procédé comprend :
    (i) préparation d'un vecteur d'expression qui incorpore une séquence d'ADN codant pour ledit peptide ou la protéine de fusion et qui est capable d'exprimer le peptide ou la protéine de fusion quand il est fourni à un hôte approprié, et
    (ii) le placement du vecteur d'expression dans l'hôte, de sorte qu'il permette l'expression du peptide ou de la protéine de fusion.

**10.** Conjugué approprié pour l'utilisation en tant que vaccin contre un RVH, comprenant un peptide tel que défini dans l'une quelconque des revendications 1 à 5, attaché à un porteur physiologiquement acceptable.

**11.** Vaccin approprié pour l'utilisation contre un RVH, lequel vaccin comprend un peptide tel que défini dans l'une quelconque des revendications 1 à 5, une protéine de fusion telle que définie dans la revendication 6 ou 7, ou un conjugué tel que défini dans la revendication 10, formulé avec un porteur ou un diluant pharmaceutiquement acceptable.

# EP 0 287 395 B1

**Revendications pour l'Etat contractant suivant : ES**

1. Procédé pour la préparation d'un peptide synthétique approprié pour l'utilisation en tant que vaccin contre un rhinovirus humain (RVH), lequel peptide est composé de jusqu'à 50 acides aminés et présente un épitope comprenant les résidus acide aminé 156 à 165 de VP2 du RVH de type 2 ou des résidus acide aminé équivalents d'un autre RVH, facultativement un ou plusieurs des résidus sont remplacés par un ou plusieurs autres résidus acide aminé qui n'affectent pas la fonction antigénique du peptide; lequel procédé comprend la synthèse chimique du peptide à partir d'acides aminés uniques et/ou de peptides préformés de deux ou plusieurs résidus acide aminé.

2. Procédé suivant la revendication 1, où le peptide est composé de jusqu'à 20 résidus acide aminé.

3. Procédé suivant la revendication 1, où le peptide est composé des résidus acide aminé de l'épitope et de jusqu'à 15 acides aminés supplémentaires ajoutés à l'extrémité C-terminale de celui-ci.

4. Procédé suivant l'une quelconque des revendications précédentes, où le peptide comprend un résidu cystéine non naturel additionnel à l'une ou aux deux extrémités.

5. Procédé suivant l'une quelconque des revendications précédentes, où le peptide incorpore une séquence porteur hétérologue pour l'épitope.

6. Procédé suivant l'une quelconque des revendications 1 à 4, comprenant de plus la liaison du peptide ainsi produit à un porteur physiologiquement acceptable.

7. Procédé pour la préparation d'un peptide synthétique approprié pour l'utilisation en tant que vaccin contre un rhinovirus humain (RVH) lequel peptide est composé de jusqu'à 50 acides aminés et présente un épitope contenant les résidus acide aminé 156 à 165 de VP2 du RVH de type 2 ou des résidus acide aminé équivalents d'un autre RVH, facultativement un ou plusieurs des résidus acide aminé étant remplacés par un ou plusieurs autres résidus acide aminé qui n'affectent pas la fonction antigénique du peptide; ou une protéine de fusion dans laquelle le peptide est attaché à une séquence porteur hétérologue à celui-ci; lequel procédé comprend :
   (i) la préparation et l'expression d'un vecteur qui incorpore une séquence d'ADN codant le peptide ou la protéine de fusion et qui est capable d'exprimer le peptide ou la protéine de fusion quand il est fourni à un hôte approprié, et
   (ii) le placement du vecteur d'expression dans l'hôte, de sorte qu'il permette l'expression du peptide ou de la protéine de fusion.

8. Procédé suivant la revendication 7, où la protéine est composée de jusqu'à 20 résidus acide aminé.

9. Procédé suivant la revendication 7, où le peptide est composé des résidus acide aminé de l'épitope et de jusqu'à 15 acides aminés supplémentaires ajoutés à l'extrémité C de celui-ci.

10. Procédé suivant l'une quelconque des revendications 7 à 9, où la séquence porteur est l'antigène de l'hépatite B (HBcAg) à l'extrémité amino duquel est attachée la séquence du peptide.

11. Procédé pour la préparation d'un vaccin, lequel procédé comprend la formulation avec un porteur ou un diluant pharmaceutiquement acceptable, du produit du procédé de l'une quelconque des revendications précédentes.

14

EP 0 287 395 B1

VP2

```
                 20                 40                 60
Rhino2   SPTVEACGYSDRIIQITRGDSTITSQDVANAIVAYGVWPHYLSSKDASAIDKPSQPDTSSNRFYTLRSVTWSSS-SKGWW
Rhino14  SPNVEACGYSDRVQQITLGNSTITTQEAAHAVVCYAEWPEYLPDVDASDVNKTSKPDTSVCRFYTLDSKTW-TTGSKGWC

         80                100               120               140
Rhino2   WKLPDALKDMGIFGENMFYHYLGRSGYTIHVQCNASKFHQGTLIVALIPEHQIASALHGNVNVGYNYTHPGETGREVKAE
Rhino14  WKLPDALKDMGVFGQNMFFHSLGRSGYTVHVQCNATKFHSGCLLVVVIPEHQLASHEGGNVSVKYTFTHPGERGIDLSSA

         160               190               200               220
Rhinn2   TRLNPDLQPTEEYWLNF-DGTLLGNITIFPHQFINLRSNNSATIIAPYVNAVPMDSMRSHNNWSLVIIPICPLE-TSSAI
Rhino14  ---NEVG-GPVKDVIYNMNGTLLGNLLJFPHQFINLRTNNTATIVIPYINSVPIDSMTRHNNVSLMVIPIAPLTYPIGAT

         240               260
Rhino2   NTIPITISISPMCAEFSGAR----AKRQ
Rhino14  PSLPITVTIAPMCTEFSGIRSXSIVP-Q
```

Figure 1

FIGURE 2

Figure 3

1     2    3   4    5

— VP 1

— VP 2
— VP 3

Figure 4